# FASCICULE DE BREVET EUROPEEN

(11) **EP 0 607 722 B1**
(45) Date de publication et mention de la délivrance du brevet: **12.03.1997**
(21) Numéro de dépôt: 93403158.4
(22) Date de dépôt: 23.12.1993
(51) Int. Cl.: C07C 45/71, C07C 45/82, C07C 47/198

(54) **Procédé continu de fabrication industrielle du diméthoxyéthanal**
Verfahren zur industriellen Herstellung von Dimethoxyethanal
Process for the industrial production of dimethoxyethanal

(30) Priorité: 19.01.1993 FR 9300470
(43) Date de publication de la demande: 27.07.1994
(73) Titulaire: SOCIETE FRANCAISE HOECHST Société anonyme dite:, F-92800 Puteaux (FR)
(72) Inventeur: Dressaire, Gilles, F-60350 Trosly Breuil (FR); Schouteeten, Alain, F-95460 Ezanville (FR)
(74) Mandataire: Rinuy, Santarelli

(56) Documents cités:
- EP-A- 0 249 530
- BULLETIN DE LA SOCIETE CHIMIQUE DE FRANCE no. 1 , 1988 , PARIS FR pages 95 - 100 A. STAMBOULI ET AL. 'Les dialkoxy - 2,2 éthanals, synthons difonctionnels à deux carbones : préparation par acétalisation du glyoxal et quelques applications en synthèse.'

## Description

La présente invention concerne un procédé continu de fabrication industrielle du diméthoxyéthanal.

Le diméthoxyéthanal, désigné ci-après DME, est un synthon de formyloléfination connu autorisant l'accès à des produits doués de propriétés physiologiques ou aromatiques intéressantes (E.J. COREY et al, J.Amer.Chem.Soc., 1988, 110, 649-650 ; A.STAMBOULI et al, Tetrahedron Letters, 1987, 27, 5301-02 ; brevets des Etats Unis d'Amérique N° 4.171.318 et 4.011.233 ; brevet suisse N° 590.857 ; brevet allemand N° 2.418.142, demande de brevet européen N° 0.246.646).

Le DME peut être préparé notamment, soit par réduction de l'ozonide du diméthylacétal de l'acroléine (demande de brevet européen N° 0.146.784), soit par clivage oxydant du dihydroxy-2,3 tétraméthoxy-1,1,4,4, butane (L.A YANOVSKAYA et al, Izv. Akad.Nauk SSSR, Otd. Khim. Nauk, 1963, 857-65), soit par hydrolyse ménagée de l'acétoxy-1 triméthoxy-1,2,2 éthane (A.STAMBOULI et al, Tetrahedron Letters , 1986, 26, 4149-52), soit par monoacétalisation du glyoxal (demande de brevet européen N° 0.249.530, A.STAMBOULI et al, Bull.Soc.Chim. France, 1988, 95-100, et H.SANGSARI et al, Synthetic Comm. 1988, 18, 1343). Ces procédés sont toutefois difficiles à mettre en oeuvre pour une production industrielle en grande quantité du fait qu'ils sont, soit dangereux (risque d'explosion de l'ozonide du diméthylacétal de l'acroléine, cf demande de brevet allemand N° 2.514.001), soit onéreux en raison de l'emploi de matières premières coûteuses ou de la nécessité d'effectuer en fin de réaction des purifications délicates par distillation.

Afin d'obvier à ces inconvénients, la demanderesse a découvert un procédé continu, industriel, de préparation du DME à partir du glyoxal en solution aqueuse.

Selon l'invention, on obtient en continu, une solution aqueuse contenant pondéralement plus de 60 % de DME, de pureté supérieure à 98,5 % avec un rendement quasi quantitatif par rapport au glyoxal mis en oeuvre par un procédé caractérisé par le fait que l'on fait réagir, en continu, du glyoxal en solution aqueuse à 70 % en poids environ avec de 8 à 12 moles de méthanol par mole de glyoxal mis en oeuvre, en présence d'une résine échangeuse de cations, à groupements sulfoniques sous forme acide, puis que la solution réactionnelle neutre obtenue, contenant pondéralement moins de 1,5 % de glyoxal, est soumise à une première distillation, à la pression atmosphérique, pour récupérer plus de 90 % du méthanol non transformé qui est recyclé, puis à une deuxième distillation sous une pression inférieure à 10 ± 2kPa pour isoler un mélange aqueux contenant la majeure partie du DME et du tétraméthoxy-1,1,2,2 éthane (désigné ci-après TME) formés, que ce mélange, après dilution avec de l'eau de manière à ce que le rapport molaire eau sur TME soit de 29 ± 4, est soumis à une distillation sous une pression inférieure à 19kPa pour éliminer dans les fractions de tête un mélange aqueux contenant plus de 98,5 % du TME présent et obtenir ainsi une solution aqueuse de DME de pureté supérieure à 98,5 % qui est, si désiré, concentrée sous pression réduite afin d'obtenir la concentration souhaitée en DME, et que, parallèlement à ces opérations, on traite en continu, à chaud, en présence d'une résine échangeuse de cations à groupements sulfoniques sous forme acide, le mélange aqueux contenant le TME ainsi que le pied de distillation issu de la colonne de distillation du mélange aqueux contenant la majeure partie du DME et du TME, pour obtenir un mélange eau-méthanol-glyoxal duquel on évapore le méthanol qui est recyclé avec le méthanol récupéré précédemment, avant d'être pour moitié recyclé avec la solution aqueuse de glyoxal de départ après avoir amené sa concentration en glyoxal à 70 % en poids environ par évaporation sous pression réduite, et pour moitié recyclé avec le mélange aqueux contenant le TME.

Dans des conditions préférentielles de mise en oeuvre de l'invention, le procédé continu ci-dessus décrit est réalisé de la manière suivante :
A) La réaction du glyoxal avec le méthanol est effectuée en continu, à la pression atmosphérique :
   - dans une colonne thermostatée à une température de 60 ± 5°C;
   - par percolation d'une mmole environ de glyoxal en solution aqueuse par x heure et par y ml d'une résine échangeuse de cations, à groupements sulfoniques sous forme acide, présentant une capacité d'échange de 3 ± 2 meq/ml, x et y étant deux nombres positifs, inférieurs à 10 tels que xy = 1, avantageusement x = y = 1 .
B) La purification de la solution réactionnelle est effectuée:
   - en continu, à pH neutre ;
   - en isolant en premier lieu, par évaporation le méthanol non transformé qui est ensuite recyclé, puis par distillation sous une pression inférieure à 10 ± 2kPa et à une température d'environ 75°C, plus de 75 % en poids du DME et du TME formés en mélange avec de l'eau et le méthanol résiduel et en récupérant simultanément dans le pied de distillation le glyoxal non transformé ainsi que le DME et le TME résiduels;
   - en diluant le distillat précédent contenant la majeure partie du DME formé avec de l'eau de manière à ce que le rapport molaire eau sur TME soit d'environ 29 ± 1, puis en le soumettant à une distillation sous pression réduite, à une température inférieure à 70 ± 5°C, pour récupérer 99 ± 1 % du TME présent en mélange avec de l'eau et le méthanol résiduel;
   - en isolant le TME formé sous la forme d'un mélange aqueux contenant pondéralement environ de 50 à 75 % d'eau ;
   - en décomposant à chaud, en présence d'une résine échangeuse de cations à groupements sulfoniques sous forme acide, le TME en solution aqueuse isolé précédemment en un mélange eau-méthanol-glyoxal duquel on sépare le méthanol par évaporation qui est ensuite recyclé, puis, sur la moitié de la solution résiduelle, l'eau en excès de manière à obtenir une solution aqueuse de glyoxal à 70 % en poids environ qui est également recyclée, l'autre moitié étant recyclée dans le milieu de décomposition ;
   - en effectuant la décomposition précédente dans une colonne identique à celle utilisée pour effectuer la réaction du glyoxal avec le méthanol, thermostatée à une température de 80 ± 5°C et remplie de la même quantité de la même résine échangeuse de cations ;
   - en introduisant dans la colonne de décomposition du TME, d'une part le pied de distillation contenant le glyoxal non transformé ainsi que du DME et du TME résiduels et d'autre part, la moitié du milieu d'hydrolyse issu de cette décomposition après récupération du méthanol formé par évaporation.

Le DME pur est isolable de sa solution aqueuse par évaporation de l'eau sous pression réduite en présence éventuellement d'un tiers solvant organique donnant un azéotrope avec l'eau suivie, si désiré, d'une distillation sous pression réduite. Le DME est un liquide incolore, facilement polymérisable, distillant à 59 ± 2°C sous une pression de 5,3 ± 0,3 kPa.

L'exemple suivant illustre la présente invention sans toutefois la limiter. Sauf indication contraire, les pourcentages indiqués sont des pourcentages pondéraux.

### EXEMPLE 1

Dans une colonne R1, équipée d'une double enveloppe, de 80 cm de diamètre et de 250 cm de longueur, thermostatée à 60 ± 3°C par une circulation d'eau chaude dans l'enceinte extérieure et contenant 1000 l d'une résine échangeuse de cations à groupements sulfoniques sous forme acide, d'une capacité d'échange de 3 ± 2 meq/ml, on fait circuler, à la pression atmosphérique et à vitesse constante, 467 kg/h d'un mélange, contenant en proportions molaires, 83,45 % de méthanol, 9,60 % d'eau et 6,95 % de glyoxal. En sortie de colonne, la solution réactionnelle neutre, contenant pondéralement moins de 1,5 % de glyoxal non transformé, est distillée dans une colonne C1 pour récupérer environ 92% du méthanol présent qu'on recueille en mélange avec 2 à 5 % d'eau, puis elle est distillée, sous une pression de 11 kPa, dans un évaporateur C2 où l'on recueille les fractions de tête contenant les acétals de glyoxal, le méthanol résiduel et moins de 0,05 % du glyoxal, lesquelles, après dilution en continu avec 125 ± 30 kg/h d'eau sont de nouveau distillées sous une pression de 17 kPa dans une colonne C3 où l'on recueille en tête de colonne un mélange aqueux contenant le méthanol et environ 99 % du TME résiduels et en pied de colonne 430 moles/h de DME de pureté supérieure à 98,75 % en solution aqueuse, qui si désiré, peut être concentrée dans une colonne C4 pour obtenir une solution aqueuse contenant 70 % de DME d'une pureté supérieure à 98,75%.

Les fractions de tête de la colonne C3 sont mélangées en continu avec d'une part, le pied de la colonne C2 et d'autre part, 50 % du pied de la colonne C5 indiquée ci-après, puis le mélange est envoyé dans une colonne R2, identique à la colonne R1, contenant la même quantité de la même résine échangeuse de cations sous forme acide et thermostatée à 85 ± 2°C par une circulation d'eau chaude. En sortie de colonne R2, on obtient un mélange ne contenant que de l'eau, du glyoxal et du méthanol qui est distillé dans une colonne C5 pour séparer en tête plus de 99 % du méthanol présent en mélange avec 1 à 5 % d'eau et en pied de colonne un mélange de glyoxal et d'eau qui est pour moitié recyclé dans la colonne R2 et pour moitié recyclé dans la colonne R1 après avoir été concentré dans une colonne C6 pour obtenir une solution aqueuse de glyoxal à 70 % on recycle ainsi environ 565 moles/h de glyoxal. Les mélanges méthanol-eau recueillis en tête des colonnes C1 et C5 sont réunis puis distillés dans une colonne C7 où l'on recueille en tête de colonne environ 11,1 kmole/h de méthanol qui sont recyclés dans la colonne R1. On obtient ainsi, en continu, du DME de pureté supérieure à 98,75 % en solution aqueuse avec un rendement supérieur à 99% par rapport au glyoxal consommé.

En marche continue, la colonne C6 est alimentée avec environ 63 kg/h d'une solution aqueuse de glyoxal à 40% en poids, soit environ 434 moles/h, et la colonne C7 est alimentée avec environ 29 kg/h (905 moles/h) de méthanol et l'on obtient environ 430 moles/h de DME en solution aqueuse qui peut être soit diluée avec de l'eau, soit concentrée sous pression réduite, afin d'obtenir la concentration souhaitée en DME.

## Revendications

1. Procédé de préparation continu du diméthoxyéthanal caractérisé par le fait que l'on fait réagir, en continu, dans une colonne (R1), du glyoxal en solution aqueuse à 70 % en poids environ avec de 8 à 12 moles de méthanol par mole de glyoxal mis en oeuvre, en présence d'une résine échangeuse de cations, à groupements sulfoniques sous forme acide, puis que la solution réactionnelle neutre obtenue, contenant pondéralement moins de 1,5 % de glyoxal, est soumise à une première distillation, dans une colonne (C1), à la pression atmosphérique, pour récupérer plus de 90 % du méthanol non transformé qui est recyclé, puis à une deuxième distillation dans une colonne (C2) sous une pression inférieure à 10 ± 2kPa pour isoler en tête un mélange aqueux contenant la majeure partie du diméthoxyéthanal et du tétraméthoxy-1,1,2,2 éthane formés, que ce mélange, après dilution avec de l'eau de manière à ce que le rapport molaire eau sur tétraméthoxy-1,1,2,2 éthane soit de 29 ± 4, est soumis à une distillation dans une colonne (C3) sous une pression inférieure à 19kPa pour éliminer dans les fractions de tête un mélange aqueux contenant plus de 98,5 % du tétraméthoxy-1,1,2,2 éthane présent et obtenir ainsi en pied une solution aqueuse de diméthoxyéthanal de pureté supérieure à 98,5 % qui est, si désiré, concentrée sous pression réduite afin d'obtenir la concentration souhaitée en diméthoxyéthanal, et que, parallèlement à ces opérations, on traite en continu, à chaud, en présence d'une résine échangeuse de cations à groupements sulfoniques sous forme acide dans une colonne (R2), le mélange aqueux contenant le tétraméthoxy-1,1,2,2 éthane provenant de la colonne (C3) ainsi que le pied de distillation issu de la colonne (C2) de distillation du mélange aqueux contenant la majeure partie du diméthoxyéthanal et du tétraméthoxy-1,1,2,2 éthane, pour obtenir un mélange eau-méthanol-glyoxal duquel on évapore dans une colonne (C5) le méthanol qui est recyclé avec le méthanol récupéré précédemment, avant d'être pour moitié recyclé avec la solution aqueuse de glyoxal de départ après avoir amené sa concentration en glyoxal à 70 % en poids environ par évaporation sous pression réduite dans une colonne (C6), et pour moitié recyclé dans une colonne (R2) avec le mélange aqueux contenant le tétraméthoxy-1,1,2,2 éthane.

2. Procédé selon la revendication 1, caractérisé par le fait qu'il est effectué par percolation d'une mmole de glyoxal en solution aqueuse, par x heure et par y ml d'une résine échangeuse de cations, à groupements sulfoniques, sous forme acide, présentant une capacité d'échange de 3 ± 2 meq/ml, x et y représentant deux nombres positifs, inférieurs à 10 tels que xy = 1.

3. Procédé selon l'une quelconque des revendications 1 et 2 caractérisé par le fait qu'il est effectué par percolation en présence de 1 ml d'une résine échangeuse de cations, à groupements sulfoniques, sous forme acide, présentant une capacité d'échange de 3 ± 2 meq/ml, par heure et par mmole de glyoxal mis en oeuvre.

4. Procédé selon l'une quelconque des revendications 1 à 3, caractérisé par le fait que la réaction du glyoxal en solution aqueuse avec le méthanol est effectuée à une température de 60 ± 5°C.

5. Procédé selon l'une quelconque des revendications 1 à 4 , caractérisé par le fait que la purification de la solution aqueuse brute de réaction du glyoxal en solution aqueuse avec le méthanol est effectuée en éliminant successivement le méthanol non transformé, le glyoxal résiduel et enfin le tétraméthoxy-1,1,2,2 éthane formé.

6. Procédé selon l'une quelconque des revendications 1 à 5, caractérisé par le fait que le tétraméthoxy-1,1,2,2 éthane est éliminé de la solution réactionnelle par distillation d'un mélange eau-tétraméthoxy-1,1,2,2 éthane.

7. Procédé selon l'une quelconque des revendications 1 à 6, caractérisé par le fait que le tétraméthoxy-1,1,2,2 éthane formé est décomposé en milieu aqueux en glyoxal et méthanol, en présence d'une résine échangeuse de cations à groupements sulfoniques sous forme acide.

8. Procédé selon l'une quelconque des revendications 1 à 7, caractérisé par le fait qu'avant l'élimination du tétraméthoxy-1,1,2,2 éthane de la solution réactionnelle, cette dernière est diluée avec de l'eau de manière à ce que le rapport molaire eau sur tétraméthoxy-1,1,2,2 éthane soit de 29 ± 4.

## Patentansprüche

1. Verfahren zur kontinuierlichen Herstellung von Dimethoxyethanal, dadurch gekennzeichnet, daß man kontinuierlich in einer Säule (R1) Glyoxal in wäßriger Lösung von etwa 70 Gew.-% mit 8 bis 12 Mol Methanol pro Mol verwendetem Glyoxal in Gegenwart eines Kationenaustauscherharzes mit Sulfonsäuregruppen in Säureform umsetzt, daß dann die erhaltene neutrale Reaktionslösung, die weniger als 1,5 Gew.-% Glyoxal enthält, einer ersten Destillation in einer Kolonne (C1) bei Atmosphärendruck unterworfen wird, um mehr als 90 % des nicht umgesetzten Methanols zurückzugewinnen, das zurückgeführt wird, dann einer zweiten Destillation in einer Kolonne (C2) unter einem Druck von weniger als 10 ± 2 kPa unterworfen wird, um am Kopf eine wäßrige Mischung zu isolieren, die den Hauptanteil an gebildetem Dimethoxyethans und 1,1,2,2-Tetramethoxyethan enthält, daß diese Mischung nach Verdünnung mit Wasser in solcher Weise, daß das Mol-Verhältnis von Wasser zu 1,1,2,2-Tetramethoxyethan 29 ± 4 beträgt, einer Destillation in einer Kolonne (C3) unter einem Druck von weniger als 19 kPa unterworfen wird, um in den Kopffraktionen eine wäßrige Mischung zu entfernen, die mehr als 98,5 % des vorliegenden 1,1,2,2-Tetramethoxyethans enthält, und in dieser Weise am Fuß eine wäßrige Lösung von Dimethoxyethanal einer Reinheit von mehr als 98,5 % zu erhalten, die auf Wunsch unter vermindertem Druck konzentriert wird, um die gewünschte Dimethoxyethanalkonzentration zu erhalten, und daß man parallel zu diesen Arbeitsgängen in der Wärme in Gegenwart eines Kationenaustauscherharzes mit Sulfonsäuregruppen in Säureform in einer Säule (R2) die das aus der Kolonne (C3) stammende 1,1, 2,2-Tetramethoxyethan enthaltende wäßrige Mischung sowie den Sumpf, der aus der Kolonne (C2) der Destillation der wäßrigen Mischung austritt, die den Hauptanteil an Dimethoxyethanal und 1,1,2,2-Tetramethoxyethan enthält, kontinuierlich behandelt, um eine Wasser/Methanol/Glyoxal-Mischung zu erhalten, von der in einer Kolonne (C5) das Methanol abgedampft wird, das mit dem vorher zurückgewonnenen Methanol zurückgeführt wird, bevor sie zur einen Hälfte mit der wäßrigen Glyoxalausgangslösung zurückgeführt wird, nachdem man ihre Glyoxalkonzentration durch Eindampfen in einer Kolonne (C6) unter vermindertem Druck auf etwa 70 Gew.-% gebracht hat, und zur anderen Hälfte mit der das 1,1, 2,2-Tetramethoxyethan enthaltenden wäßrigen Mischung in eine Säule (R2) zurückgeführt wird.

2. Verfahren nach Anspruch 1, dadurch gekennzeichnet, daß es durch Perkolation von 1 mmol Glyoxal in wäßriger Lösung pro x Stunde und pro y ml eines Kationenaustauscherharzes mit Sulfonsäuregruppen in Säureform, das eine Austauschkapazität von 3 ± 2 meq/ml aufweist, durchgeführt wird, wobei x und y zwei positive Zahlen kleiner als 10 bedeuten, so daß xy = 1 ist.

3. Verfahren nach einem der Ansprüche 1 und 2, dadurch gekennzeichnet, daß es durch Perkolation in Gegenwart von 1 ml eines Kationenaustauscherharzes mit Sulfonsäuregruppen in Säureform, das eine Austauschkapazität von 3 ± 2 meq/ml aufweist, pro Stunde und pro mmol verwendetem Glyoxal durchgeführt wird.

4. Verfahren nach einem der Ansprüche 1 bis 3, dadurch gekennzeichnet, daß die Reaktion von Glyoxal in wäßriger Lösung mit dem Methanol bei einer Temperatur von 60 ± 5°C durchgeführt wird.

5. Verfahren nach einem der Ansprüche 1 bis 4, dadurch gekennzeichnet, daß die Reinigung der rohen wäßrigen Lösung aus der Reaktion von Glyoxal in wäßriger Lösung mit dem Methanol durch aufeinanderfolgende Entfernung des nicht umgesetzten Methanols, des restlichen Glyoxals und schließlich des gebildeten 1,1,2,2-Tetramethoxyethans durchgeführt wird.

6. Verfahren nach einem der Ansprüche 1 bis 5, dadurch gekennzeichnet, daß das 1,1,2,2-Tetramethoxyethan aus der Reaktionslösung durch Destillation einer Wasser/1,1,2,2-Tetramethoxyethan-Mischung entfernt wird.

7. Verfahren nach einem der Ansprüche 1 bis 6, dadurch gekennzeichnet, daß das gebildete 1,1,2,2-Tetramethoxyethan in wäßrigem Medium in Gegenwart eines Kationenaustauscherharzes mit Sulfonsäuregruppen in Säureform zu Glyoxal und Methanol zersetzt wird.

8. Verfahren nach einem der Ansprüche 1 bis 7, dadurch gekennzeichnet, daß vor dem Entfernen von 1,1,2,2-Tetramethoxyethan aus der Reaktionslösung diese letztere mit Wasser in solcher Weise verdünnt wird, daß das Mol-Verhältnis von Wasser zu 1,1, 2,2-Tetramethoxyethan 29 ± 4 beträgt.

## Claims

1. Process for the continuous preparation of dimethoxy ethanal characterized in that an aqueous solution of glyoxal at 70 % by weight is continuously reacted, in a column (R1), with 8 to 12 moles of methanol per mole of glyoxal used, in the presence of a cation exchange resin having sulphonic groups in acid form, then the neutral reaction solution obtained, containing less than 1.5 % by weight of glyoxal, is subjected to an initial distillation, in a column (C1), at atmospheric pressure, in order to recover more than 90 % of the unreacted methanol, which is recycled, then to a second distillation in a column (C2) under a pressure of less than 10 ± 2 kPa in order to isolate from the top an aqueous mixture containing the larger portion of the dimethoxy ethanal and of the 1,1,2,2-tetramethoxyethane formed, that this mixture, after dilution with water in a manner such that the molar ratio of water to 1,1,2,2-tetramethoxyethane is 29 ± 4, is subjected to a distillation in a column (C3) under a pressure of less than 19 kPa in order to eliminate, from the top fractions, an aqueous mixture containing more than 98.5 % of the 1,1,2,2-tetramethoxyethane present and thus to obtain at the bottom an aqueous solution of dimethoxy ethanal having a purity greater than 98.5 %, which, if desired, is concentrated under reduced pressure in order to obtain the required concentration of dimethoxy ethanal, and that, at the same time as these operations, the aqueous mixture containing the 1,1,2,2-tetramethoxyethane originating from column (C3) as well as the bottom fraction of the distillation produced from column (C2) for the distillation of the aqueous mixture containing the larger portion of the dimethoxy ethanal and of the 1,1,2,2-tetramethoxyethane is treated continuously while warm in the presence of a cation exchange resin having sulphonic groups in acid form in a column (R2), in order to obtain a water-methanol-glyoxal mixture, the methanol of which is evaporated off in a column (C5) and recycled with the methanol recovered previously, before half is recycled with the starting aqueous glyoxal solution, after having adjusted its concentration of glyoxal to approximately 70 % by weight by evaporation under reduced pressure in a column (C6), and half is recycled in a column (R2) with the aqueous mixture containing the 1,1,2,2-tetramethoxyethane.

2. Process according to claim 1, characterized in that it is carried out by percolating one mmole of glyoxal in aqueous solution, per x hours and per y ml of a cation exchange resin, having sulphonic groups, in acid form, having an exchange capacity of 3 ± 2 meq/ml, x and y representing two positive numbers, lower than 10 such that xy = 1.

3. Process according to any one of claims 1 and 2, characterized in that it is carried out by percolating in the presence of 1 ml of a cation exchange resin, having sulphonic groups, in acid form, having an exchange capacity of 3 ± 2 meq/ml, per hour and per mmole of glyoxal used.

4. Process according to any one of claims 1 to 3, characterized in that the reaction of the glyoxal in aqueous solution with the methanol is effected at a temperature of 60 ± 5°C.

5. Process according to any one of claims 1 to 4, characterized in that purification of the crude aqueous solution for the reaction of the glyoxal in aqueous solution with the methanol is carried out by successively removing the unreacted methanol, the residual glyoxal and finally the 1,1,2,2-tetramethoxyethane formed.

6. Process according to any one of claims 1 to 5, characterized in that the 1,1,2,2-tetramethoxyethane is removed from the reaction solution by distilling a water-1,1,2,2-tetramethoxyethane mixture.

7. Process according to any one of claims 1 to 6, characterized in that the 1,1,2,2-tetramethoxyethane formed is decomposed in aqueous medium into glyoxal and methanol, in the presence of a cation exchange resin having sulphonic groups in acid form.

8. Process according to any one of claims 1 to 7, characterized in that, before removing the 1,1,2,2-tetramethoxyethane from the reaction solution, the latter is diluted with water in such a manner that the molar ratio of water to 1,1,2,2-tetramethoxyethane is 29 ± 4.
